# EUROPÄISCHE PATENTSCHRIFT

(11) **EP 1 399 231 B1**
(45) Veröffentlichungstag und Bekanntmachung des Hinweises auf die Patenterteilung: **02.02.2005**
(21) Anmeldenummer: 02748752.9
(22) Anmeldetag: 10.06.2002
(51) Int. Cl.: B01D 1/00

(54) **VERFAHREN ZUR ENTFERNUNG VON AMEISENSÄURE AUS WÄSSRIGEN LÖSUNGEN**
METHOD FOR REMOVING FORMIC ACID FROM AQUEOUS SOLUTIONS
PROCEDE D'EXTRACTION D'ACIDE FORMIQUE DANS DES SOLUTIONS AQUEUSES

(30) Priorität: 11.06.2001 DE 10128249
(43) Veröffentlichungstag der Anmeldung: 24.03.2004
(73) Patentinhaber: BASF AKTIENGESELLSCHAFT, 67056 Ludwigshafen (DE)
(72) Erfinder: NÖBEL, Thomas, 67133 Maxdorf (DE)
(74) Vertreter: Isenbruck, Günter, Dr.
(86) Internationale Anmeldenummer: PCT/EP2002/006336
(87) Internationale Veröffentlichungsnummer: WO 2002/100502

(56) Entgegenhaltungen:
- WO-A-97/31882
- SU-A- 1 033 431
- DR. FRIEDHELM BÖKELMANN: "Ullmanns Encyklopädie der technischen Chemie; Cyclohexanol" 1975 , VERLAG CHEMIE , WEINHEIM/BERGSTR. XP002219653 9 Absatz 3.2 Flüssigphasenoxidation und Absatz 10. Literatur Seite 690 -Seite 695

## Beschreibung

Die Erfindung betrifft ein Verfahren zur zumindest teilweisen Entfernung von Ameisensäure aus einer Ameisensäure, Cyclohexanon und Cyclohexanol als wesentliche organische Komponenten enthaltenden wässrigen Lösung.

Cyclohexanon und Cyclohexanol, die zu epsilon-Caprolactam weiterverabreitet werden, werden großtechnisch durch Cyclohexan-Oxidation mit Sauerstoff oder einem Sauerstoff enthaltenden Gas erhalten. Dabei fällt ein komplexes Produktgemisch an, das außer den Hauptwertprodukten Cyclohexanon und Cyclohexanol noch 1,2- und 1,4-Cyclohexandiol, Adipinsäure, 6-Hydroxycapronsäure, Glutarsäure, 5-Hydroxyvaleriansäure, Ameisensäure sowie eine Vielzahl weiterer oxigenierter Komponenten als Nebenprodukte enthält.

Durch Extraktion dieses Produktgemischs mit einer wässrigen Waschflüssigkeit werden die genannten Cyclohexandiole, Mono- und Dicarbonsäuren von den Hauptwertprodukten Cyclohexanon und Cyclohexanol abgetrennt, wobei eine wässrige Lösung der Cyclohexandiole, Mono- und Dicarbonsäuren und weiterer organischer Komponenten erhalten wird. Diese enthält außerdem geringe Mengen der Wertprodukte Cyclohexanon und Cyclohexanol. Die als Dicarbonsäurelösung bezeichnete wässrige Lösung wird anschließend in einer Kolonne destillativ entwässert, wobei als Kopfprodukt ein wässriger Strom anfällt, der als wesentliche organische Komponenten Ameisensäure, Cyclohexanon und Cyclohexanol enthält.

Aus dem entwässerten Carbonsäuregemisch wird durch Veresterung, Hydrierung und Destillation 1,6-Hexandiol gewonnen. Das wässrige Kopfprodukt, enthaltend die Ameisensäure, Cyclohexanon und Cyclohexanol, wurde bisher verworfen, beispielsweise in eine Kläranlage eingeleitet oder verbrannt. Eine Weiterverwertung der Cyclohexanon, Cyclohexanol und Ameisensäure enthaltenden wässrigen Lösung, beispielsweise durch Abtrennung von Cyclohexanon und Cyclohexanol und deren Weiterverarbeitung zu epsilon-Caprolactam, schied bisher wegen der durch die Ameisensäure bedingten Korrosionsprobleme aus.

Aus R. S. Coffey, Chemical Communications, 1967, S. 923 - 24 ist bekannt, dass sich Ameisensäure an Phosphin-Komplexen der Edelmetalle Rh, Ru, Ir und Pt zu CO₂ und H₂ zersetzen lässt.

SU-A 1 033 431 beschreibt die Zersetzung von Ameisensäure zu CO und H₂O an einem Katalysator aus CaO und P₂O₅ bzw. CaO, P₂O₅ und B₂O₃.

JP-A 1222917 beschreibt die Zersetzung von Ameisensäure an Oxiden und Hydroxiden von Zirkon, Titan, Aluminium und Eisen, die mit einer Sulfatlösung behandelt und anschließend calciniert worden sind.

Aufgabe der Erfindung ist es, ein einfaches und kostengünstiges Verfahren bereitzustellen, um die bei der destillativen Entwässerung von Carbonsäurelösungen, die bei der Wasserextraktion des Produktgemisches der Cyclohexan-Oxidation erhalten werden, anfallenden, Cyclohexanon, Cyclohexanol und Ameisensäure enthaltenden wässrigen Lösungen aufzuarbeiten und einer Wiederverwertung zuzuführen.

Gelöst wird die Aufgabe durch ein Verfahren zur zumindest teilweisen Entfernung von Ameisensäure aus einer Ameisensäure, Cyclohexanon und Cyclohexanol enthaltenden wässrigen Lösung, bei dem die Ameisensäure an einem basischen Metalloxid von Metallen der Gruppen 2, 4, 5, 12 und/oder 14 oder an einem Edelmetall der Gruppen 8 - 11 auf einem oxidischen oder nicht-oxidischen Träger als Zersetzungskatalysator zersetzt wird.

Die Ameisensäure wird an den Zersetzungskatalysatoren zu gasförmigen Zersetzungsprodukten, überwiegend zu CO₂ und H₂, zersetzt.

Das erfindungsgemäße Verfahren ermöglicht es, Ameisensäure selektiv aus den wässrigen Lösungen zu entfernen, ohne dass es gleichzeitig zu einem Abbau der als Wertprodukte enthaltenen Komponenten Cyclohexanon und Cyclohexanol kommt. Dabei ist es überraschend, dass es unter den Reaktionsbedingungen nicht zu einem Verlust an Cyclohexanon durch basenkatalysierte Aldolkondensation von Cyclohexanon und auch nicht zu einem Verlust an Cyclohexanol durch Eliminierung zu Cyclohexen kommt.

Geeignete basische Metalloxide von Metallen der Gruppen 2, 4, 5, 12 und 14 sind BeO, MgO, CaO, BaO, TiO₂, ZrO₂, V₂O₅, ZnO, CdO und SnO₂. Geeignete Metalle der Gruppen 8 - 11 sind Ru, Rh, Ir, Ni, Pd, Pt, Cu, Ag und Au. Geeignete oxidische Träger sind beispielsweise Al₂O₃, TiO₂, MgO, ein geeigneter nicht-oxidischer Träger ist Aktivkohle (C).

Als Zersetzungskatalysator bevorzugt ist ZnO bzw. ein Gemisch aus ZnO und CaO oder ein vorgehend genanntes Edelmetall auf Aktivkohle.

Die Zersetzung wird üblicherweise bei einer Temperatur von 100 bis 400 °C, bevorzugt von 150 bis 350 °C, besonders bevorzugt von 170 bis 220 °C durchgeführt. Die Verweilzeit beträgt beispielsweise von 1 bis 6 h. Im allgemeinen werden dabei mindestens 90%, vorzugsweise mindestens 95%, besonders bevorzugt mindestens 98% der Ameisensäure zersetzt.

Die Zersetzung kann diskontinuierlich oder kontinuierlich an einem suspendierten oder fest angeordneten Katalysator, beispielsweise in Sumpf- oder Rieselfahrweise, erfolgen. Bevorzugt erfolgt die Zersetzung kontinuierlich, besonders bevorzugt an einem fest angeordneten Zersetzungskatalysator. Geeignete Reaktoren sind beispielsweise Rohrreaktoren mit fest angeordneter Katalysator-Schüttung.

Die Ameisensäure, Cyclohexanon und Cyclohexanol enthaltende wässrige Lösung wird üblicherweise durch Extraktion des bei der Cyclohexan-Oxidation mit Sauerstoff oder einem Sauerstoff enthaltenden Gas erhaltenen Gasgemisches mit einer wässrigen Waschflüssigkeit und Destillation der erhaltenen carbonsäurehaltigen wässrigen Lösung gewonnen.

Die dabei erhaltene wässrige Lösung, aus der die Ameisensäure erfindungsgemäß zumindest teilweise entfernt wird, enthält in der Regel
(a) 3 bis 6 Gew.-% Ameisensäure,
(b) 0,1 bis 2 Gew.-% Cyclohexanol,
(c) 0,1 bis 2 Gew.-% Cyclohexanon,
(d) 92 bis 96,8 Gew.-% Wasser.

Gegenstand der Erfindung ist auch ein Verfahren zur Weiterverarbeitung von Reaktionsprodukten der Cyclohexan-Oxidation mit den Schritten
(i) Extraktion des bei der Cyclohexan-Oxidation erhaltenen Produktgasgemischs mit einer wässrigen Waschflüssigkeit, wobei eine carbonsäurehaltige wässrige Lösung erhalten wird,
(ii) destillative Trennung der in Schritt (i) erhaltenen carbonsäurehaltigen wässrigen Lösung in eine wässrige Lösung enthaltend Ameisensäure, Cyclohexanon und Cyclohexanol neben weiteren organischen Minderkomponenten, und in ein Carbonsäuregemisch, wobei das Carbonsäuregemisch in üblicher Weise zu 1,6-Hexandiol weiterverarbeitet werden kann,
(üi) zumindest teilweise Entfernung der Ameisensäure aus der in Schritt (ii) erhaltenen Ameisensäure, Cyclohexanon und Cyclohexanol enthaltenden wässrigen Lösung durch Zersetzung der Ameisensäure an einem basischen Metalloxid von Metallen der Gruppen 2, 4, 5, 12 und/oder 14 oder an einem Edelmetall der Gruppen 8 - 11 auf einem oxidischen oder nicht oxidischen Träger als Zersetzungskatalysator,
(iv) Weiterverarbeitung der in Schritt (iii) erhaltenen Cyclohexanon und Cyclohexanol enthaltenden wässrigen Lösung zu epsilon-Caprolactam,
   oder
   Einsatz der in Schritt (iii) erhaltenen Cyclohexanon und Cyclohexanol enthaltenden wässrigen Lösung als Waschflüssigkeit in Schritt (i).

In Schritt (i) wird das bei der Cyclohexan-Oxidation erhaltene Produktgemisch mit einer wässrigen Waschflüssigkeit extrahiert, wobei eine carbonsäurehaltige wässrige Lösung erhalten wird, die üblicherweise 10 bis 40 Gew.-% Adipinsäure, 10 bis 40 Gew.-% 6-Hydroxycapronsäure, 1 bis 10 Gew.-% Glutarsäure, 1 bis 10 Gew.-% 5-Hydroxyvaleriansäure, 1 bis 5 Gew.-% 1,2-Cyclohexandiol, 1 bis 5 Gew.-% 1,4-Cyclohexandiol, 2 bis 10 Gew.-% Ameisensäure, 0,1 bis 2 Gew.-% Cyclohexanon, 0,1 bis 2 Gew.-% Cyclohexanol sowie eine Vielzahl weiterer Mono- und Dicarbonsäuren, Ester, Oxo- und Oxa-Verbindungen, deren Einzelgehalte im allgemeinen 5 Gew.-% nicht übersteigen, wie Essigsäure, Propionsäure, Buttersäure, Valeriansäure, Capronsäure, Oxalsäure, Malonsäure, Bernsteinsäure, 4-Hydroxybuttersäure und gamma-Butyrolacton enthält.

In Schritt (ii) wird die wässrige Lösung enthaltend Ameisensäure, Cyclohexanon und Cyclohexanol destillativ abgetrennt. Das verbleibende Carbonsäuregemisch kann in üblicher Weise zu 1,6-Hexandiol, beispielsweise wie in WO 97/31882 beschrieben, verarbeitet werden. Dazu werden die in dem Carbonsäuregemisch enthaltenen Mono- und Dicarbonsäuren mit einem niedermolekularen Alkohol zu den entsprechenden Carbonsäureestern umgesetzt, das erhaltene Veresterungsgemisch in einer ersten Destillationsstufe von überschüssigem Alkohol und von Leichtsiedern befreit, das Sumpfprodukt in einer weiteren Destillationsstufe von Cyclohexandiolen befreit, die Esterfraktion katalytisch hydriert und aus dem Hydrieraustrag 1,6-Hexandiol destillativ gewonnen.

Die in Schritt (iii) gewonnene, Cyclohexanon und Cyclohexanol enthaltende, an Ameisensäure abgereicherte wässrige Lösung kann zu epsilon-Caprolactam weiterverarbeitet werden. Eine solche Weiterverarbeitung ist ohne vorherige Entfernung der Ameisensäure wegen der durch diese bedingten Korrosionsprobleme nicht möglich. Zur Weiterverarbeitung werden Cyclohexanon und Cyclohexanol destillativ aus der wässrigen Lösung abgetrennt, Cyclohexanol zu Cyclohexanon oxidiert und aus Cyclohexanon in an sich bekannter Weise epsilon-Caprolactam hergestellt.

Die an Ameisensäure abgereicherte wässrige Lösung kann ferner in Schritt (i) als Waschflüssigkeit eingesetzt werden. Dadurch werden die Verluste an Cyclohexanon und Cyclohexanol durch den Extraktionsschritt (i) minimiert.

Die an Ameisensäure abgereicherte wässrige Lösung kann auch als Abwasser in eine Kläranlage eingeleitet werden. Vorteilhaft ist dabei der verringerte TOC-Gehalt des Abwassers.

Die Erfindung wird durch das nachstehende Beispiel näher erläutert.

### Beispiel

In einem 50 ml-Autoklaven werden 32,1g der ameisensäurehaltigen wässrigen Lösung vorgelegt, die durch destillative Entwässerung der durch Extraktion der Produktgase der Cyclohexan-Oxidation mit Wasser erhaltenen carbonsäurehaltigen wässrigen Lösung gewonnen wurde. Diese ameisensäurehaltige wässrige Lösung wies folgende Zusammensetzung auf:

| | |
|---|---|
| 6 Gew.-% | Ameisensäure, |
| 1 Gew.-% | Cyclohexanon, |
| 1 Gew.-% | Cyclohexanol, |
| 92 Gew.-% | Wasser. |

Das Gemisch wird mit 10 g der in untenstehender Tabelle angegebenen Zersetzungskatalysatoren in Pulverform bzw. in Form von Splitt versetzt. Das Gemisch wird über einen Zeitraum von 2 bis 4 h auf 170 bis 220 °C erhitzt, wobei aufgrund der gasförmigen Zersetzungsprodukte (CO, CO₂ und H₂) ein Druckanstieg zu verzeichnen ist. Nach Abkühlen der Reaktionsmischung wird der Ameisensäuregehalt mittels Säure/Base-Titration bestimmt. Der Cyclohexanon- und Cyclohexanol-Gehalt wurde gaschromatographisch bestimmt und wies keine Veränderung auf.

| **Säurezahl/pH** **(vorher)** | **Temperatur** **[°C]** | **Verweilzeit** **[h]** | **Druck**^{**(1)**} **[bar]** | **Säurezahl/pH** **(nachher)** |
|---|---|---|---|---|
| **Katalysator: ZnO als Stränge** | | | | |
| 75/1,0 | 170 | 2 | 15 | 42/6,0 |
| 75/1,1 | 170 | 4 | 25 | 29/6,0 |
| | | | | |
| 75/1,1 | 190 | 2 | 25 | 17/6,1 |
| 75/1,2 | 190 | 4 | 55 | < 1/6,6 |
| | | | | |
| 88/1,1 | 200 | 2 | 80 | 5,3/6,5 |
| 88/1,0 | 200 | 4 | 70 | 1,4/4,7 |
| | | | | |
| 75/1,2 | 210 | 2 | 70 | 2/6,3 |
| 88/1,0 | 220 | 2 | 70 | < 0,5/7,0 |
| 88/1,1 | 220 | 4 | 70 | < 0,5/6,9 |

| **Katalysator: 50 Gew.-% ZnO / 50 Gew.-% CaO / Calcit als Formkörper** | | | | |
|---|---|---|---|---|
| 78/1,1 | 190 | 2 | 55 | 1,5/6,2 |
| 78/1,1 | 200 | 2 | 57 | 2,4/6,4 |
| 78/1,1 | 210 | 2 | 68 | 0,9/6,2 |

| **Katalysator: 50 Gew.-% ZnO / 50 Gew.-% CaO als Formkörper** | | | | |
|---|---|---|---|---|
| 78/1,1 | 200 | 2 | 50 | < 0,5/5,8 |
| 78/1,1 | 190 | 2 | 62 | 1,2/5,9 |

| | | | | |
|---|---|---|---|---|
| (1) gemessener Enddruck | | | | |

## Patentansprüche

1. Verfahren zur zumindest teilweisen Entfernung von Ameisensäure aus einer Ameisensäure, Cyclohexanon und Cyclohexanol enthaltenden wässrigen Lösung, bei dem die Ameisensäure an einem basischen Metalloxid von Metallen der Gruppen 2, 4, 5 12 und/oder 14 oder an einem Edelmetall der Gruppen 8 - 11 auf einem oxidischen Träger als Zersetzungskatalysator zersetzt wird.

2. Verfahren nach Anspruch 1, **dadurch gekennzeichnet, dass** der Zersetzungskatalysator Zinkoxid und/oder Calciumoxid enthält.

3. Verfahren nach Anspruch 1 oder 2, **dadurch gekennzeichnet, dass** die Zersetzung bei einer Temperatur von 100 bis 400 °C durchgeführt wird.

4. Verfahren nach einem der Ansprüche 1 bis 3, **dadurch gekennzeichnet, dass** die Zersetzung kontinuierlich an dem fest angeordneten oder suspendierten Zersetzungskatalysator durchgeführt wird.

5. Verfahren nach einem der Ansprüche 1 bis 4, **dadurch gekennzeichnet, dass** die Ameisensäure, Cyclohexanon und Cyclohexanol enthaltende wässrige Lösung durch Extraktion des bei der Cyclohexan-Oxidation mit Sauerstoff oder einem Sauerstoff enthaltenden Gas erhaltenen Produktgasgemisches mit einer wässrigen Waschflüssigkeit und Destillation der so erhaltenen wässrigen Carbonsäurelösung gewonnen wird.

6. Verfahren nach einem der Ansprüche 1 bis 5, **dadurch gekennzeichnet, dass** Ameisensäure aus einer wässrigen Lösung enthaltend
(a) 3 bis 6 Gew.-% Ameisensäure,
(b) 0,1 bis 2 Gew.-% Cyclohexanol,
(c) 0,1 bis 2 Gew.-% Cyclohexanon,
(d) 92 bis 96,8 Gew.-% Wasser
entfernt wird.

7. Verfahren zur Weiterverarbeitung von Reaktionsprodukten der Cyclohexan-Oxidation mit den Schritten
(i) Extraktion des bei der Cyclohexan-Oxidation erhaltenen Produktgasgemischs mit einer wässrigen Waschflüssigkeit, wobei eine carbonsäurehaltige wässrige Lösung erhalten wird,
(ii) destillative Trennung der in Schritt (i) erhaltenen carbonsäurehaltigen wässrigen Lösung in eine wässrige Lösung enthaltend Ameisensäure, Cyclohexanon und Cyclohexanol neben weiteren organischen Minderkomponenten, und in ein Carbonsäuregemisch, wobei das Carbonsäuregemisch in üblicher Weise zu 1,6-Hexandiol weiterverarbeitet werden kann,
(iii) zumindest teilweise Entfernung der Ameisensäure aus der in Schritt (ii) erhaltenen Ameisensäure, Cyclohexanon und Cyclohexanol enthaltenden wässrigen Lösung durch Zersetzung der Ameisensäure an einem basischen Metalloxid von Metallen der Gruppen 2, 4, 5 12 und/oder 14 oder an einem Edelmetall der Gruppen 8 - 11 auf einem oxidischen oder nicht-oxidischen Träger als Zersetzungskatalysator,
(iv) Weiterverarbeitung der in Schritt (iii) erhaltenen Cyclohexanon und Cyclohexanol enthaltenden wässrigen Lösung zu epsilon-Caprolactam,
oder
Einsatz der in Schritt (iii) erhaltenen Cyclohexanon und Cyclohexanol enthaltenden wässrigen Lösung als Waschflüssigkeit in Schritt (i).

## Claims

1. A method of at least partly removing formic acid from an aqueous solution comprising formic acid, cyclohexanone and cyclohexanol, which comprises decomposing the formic acid over a basic metal oxide of metals of groups 2, 4, 5, 12 and/or 14 or over a noble metal of groups 8-11 on an oxidic support as decomposition catalyst.

2. A method as claimed in claim 1, wherein the decomposition catalyst comprises zinc oxide and/or calcium oxide.

3. A method as claimed in claim 1 or 2, wherein the decomposition is carried out at from 100 to 400°C.

4. A method as claimed in any of claims 1 to 3, wherein the decomposition is carried out continuously over a fixed-bed or suspended decomposition catalyst.

5. A method as claimed in any of claims 1 to 4, wherein the aqueous solution comprising formic acid, cyclohexanone and cyclohexanol is obtained by extraction of the product gas mixture obtained in the oxidation of cyclohexane by means of oxygen or an oxygen-containing gas using an aqueous scrubbing liquid and distillation of the aqueous carboxylic acid solution obtained in this way.

6. A method as claimed in any of claims 1 to 5, wherein formic acid is removed from an aqueous solution comprising
(a) from 3 to 6% by weight of formic acid,
(b) from 0.1 to 2% by weight of cyclohexanol,
(c) from 0.1 to 2% by weight of cyclohexanone,
(d) from 92 to 96.8% by weight of water.

7. A process for the further processing of reaction products of the oxidation of cyclohexane, which comprises the steps
(i) extraction of the product gas mixture obtained in the oxidation of cyclohexane using an aqueous scrubbing liquid to give an aqueous solution comprising carboxylic acids,
(ii) separation of the aqueous solution comprising carboxylic acids obtained in step (i) into an aqueous solution comprising formic acid, cyclohexanone and cyclohexanol together with further minor organic components and a carboxylic acid mixture by distillation, with the carboxylic acid mixture being able to be processed further in a customary manner to give 1,6-hexanediol,
(iii) at least a partial removal of the formic acid from the aqueous solution comprising formic acid, cyclohexanone and cyclohexanol obtained in step (ii) by decomposition of the formic acid over a basic metal oxide of metals of groups 2, 4, 5, 12 and/or 14 or over a noble metal of groups 8-11 on an oxidic or nonoxidic support as decomposition catalyst,
(iv) further processing of the aqueous solution comprising cyclohexanone and cyclohexanol obtained in step (iii) to give epsilon-caprolactam,
or
use of the aqueous solution comprising cyclohexanone and cyclohexanol obtained in step (iii) as scrubbing liquid in step (i).

## Revendications

1. Procédé d'élimination au moins partielle d'acide formique à partir d'une solution aqueuse contenant de l'acide formique, de la cyclohexanone et du cyclohexanol, procédé dans lequel l'acide formique est décomposé sur un oxyde métallique basique des métaux des groupes 2, 4, 5, 12 et/ou 14 ou sur un métal noble des groupes 8-11 sur un support oxydé, comme catalyseur de décomposition.

2. Procédé suivant la revendication 1, **caractérisé en ce que** le catalyseur de décomposition contient de l'oxyde de zinc et/ou de l'oxyde de calcium.

3. Procédé suivant l'une des revendications 1 et 2, **caractérisé en ce que** la décomposition est effectuée à une température de 100 à 400°C.

4. Procédé suivant l'une des revendications 1 à 3, **caractérisé en ce que** la décomposition est effectuée de manière continue sur le catalyseur de décomposition agencé de manière fixe ou en suspension.

5. Procédé suivant l'une des revendications 1 à 4, **caractérisé en ce que** la solution aqueuse contenant de l'acide formique, de la cyclohexanone et du cyclohexanol est obtenue par extraction à l'aide d'un liquide de lavage aqueux du mélange gazeux produit qui est obtenu lors de l'oxydation de cyclohexane avec de l'oxygène ou un gaz contenant de l'oxygène, et par distillation de la solution aqueuse d'acide carboxylique ainsi obtenue.

6. Procédé suivant l'une des revendications 1 à 5, **caractérisé en ce que** l'acide formique est éliminé à partir d'une solution aqueuse contenant
(a) 3 à 6% en poids d'acide formique,
(b) 0,1 à 2% en poids de cyclohexanol,
(c) 0,1 à 2% en poids de cyclohexanone,
(d) 92 à 96,8% en poids d'eau.

7. Procédé de traitement ultérieur de produits réactionnels de l'oxydation du cyclohexane, comprenant les étapes
(i) d'extraction à l'aide d'un liquide de lavage aqueux du mélange gazeux produit, qui est obtenu lors de l'oxydation de cyclohexane, une solution aqueuse contenant de l'acide carboxylique étant obtenue,
(ii) de séparation par distillation de la solution aqueuse contenant de l'acide carboxylique, obtenue dans l'étape (i), en une solution aqueuse contenant de l'acide formique, de la cyclohexanone et du cyclohexanol à côté d'autres composants organiques en moindre teneur, et en un mélange d'acides carboxyliques, le mélange d'acides carboxyliques pouvant être traité ultérieurement d'une manière courante en 1, 6-hexanediol,
(iii)d'élimination au moins partielle de l'acide formique à partir de la solution aqueuse contenant de l'acide formique, de la cyclohexanone et du cyclohexanol, obtenue dans l'étape (ii), par décomposition de l'acide formique sur un oxyde métallique basique des métaux des groupes 2, 4, 5, 12 et/ou 14 ou sur un métal noble des groupes 8-11, sur un support oxydé ou non oxydé, comme catalyseur de décomposition,
(iv) de traitement ultérieur de la solution aqueuse contenant de la cyclohexanone et du cyclohexanol obtenue dans l'étape (iii) en epsilon-caprolactame, ou
de mise en oeuvre de la solution aqueuse contenant de la cyclohexanone et du cyclohexanol, obtenue dans l'étape (iii), comme liquide de lavage dans l'étape (i).
